# EUROPÄISCHE PATENTSCHRIFT

(11) **EP 4 247 594 B1**
(45) Veröffentlichungstag und Bekanntmachung des Hinweises auf die Patenterteilung: **19.03.2025**
(21) Anmeldenummer: 21840784.9
(22) Anmeldetag: 19.11.2021
(51) Int. Cl.: A61F 5/02, B25J 9/00, A61H 3/00

(54) **HEBEHILFE UND FESTES RÜCKENELEMENT**
LIFTING AID AND RIGID BACK ELEMENT
DISPOSITIF D'AIDE AU LEVAGE ET ÉLÉMENT DORSAL FIXE

(30) Priorität: 19.11.2020 DE 202020066660 U
(43) Veröffentlichungstag der Anmeldung: 27.09.2023
(73) Patentinhaber: Heinzelmann, Dominik, 72250 Freudenstadt (DE)
(72) Erfinder: Heinzelmann, Dominik, 72250 Freudenstadt (DE)
(74) Vertreter: Neidl-Stippler, Cornelia
(86) Internationale Anmeldenummer: PCT/DE2021/100921
(87) Internationale Veröffentlichungsnummer: WO 2022/105966

(56) Entgegenhaltungen:
- WO-A1-2011/127421
- WO-A1-2014/195373
- WO-A1-2019/161232
- WO-A1-2020/224836
- AU-A1- 2018 256 552
- DE-A1- 102018 112 556
- DE-A1- 102018 112 558
- US-A1- 2018 272 524
- US-A1- 2019 358 808

## Beschreibung

Die Erfindung betrifft eine Hebehilfe und eine Verwendung derselben. Hebehilfen zur Unterstützung beim Heben von Lasten sind in den verschiedensten Formen bekannt: Sie werden zur Unterstützung der menschlichen Kraft benötigt - bspw. als Exoskelette, aber auch als mobile Trageunterstützung, die - von einer tragenden Person angelegt - diesen in seiner Tätigkeit unterstützt. Hebehilfen werden im Bauwesen, in Pflegeeinrichtungen, bei Umzugshelfern und generell zur Rückenschonung benötigt. Hebehilfen sind bspw. aus der EP3176123A1, DE10201420520A1, der DE 1020182067823A1, der DE2020111104994 U1 oder der DE 10 2004023981A1, der DE102029111718A1 der WO2019/161232A1, der US2020026854A1 und der WO2020/224836A1 bekannt. Viele bekannte Systeme waren schwer, unhandlich, kompliziert aufgebaut und/oder störanfällig und schränkten den Träger in seiner natürlichen Bewegung ein. Andere nutzen stets einen Elektromotor zur Unterstützung. Besonders elektrisch angetriebene Hebeunterstützungen mussten mit ausreichend elektrischer Energie versorgt werden, wobei Motor und Energieversorgung das Gewicht sowie den Aufwand zu ihrer Herstellung zusätzlich erhöhen - dies besonders dann, wenn der Antrieb das Beugen des Trägers nachbilden soll.

Es ist daher Aufgabe der Erfindung, eine einfache, mechanische Hebehilfe vorzuschlagen.

Die Aufgabe wird durch eine Hebehilfe mit den Merkmalen des Anspruchs 1 gelöst. Vorteilhafte Weiterbildungen ergeben sich aus den abhängigen Ansprüchen.

Die Erfindung bezieht sich also auf eine Hebehilfe mit einem starren Rückenelement 1 zur Leitung von Unterstützungskräften, das zwischen einer Oberkörperanbindung und Unterkörperanbindungen (8,8.1) sowie einem Bauchgurt 17 angebunden und gehalten ist, wobei mindestens ein erster mechanischer Energiespeicher (21, 21.1) für die Unterstützung an der Unterkörperanbindung (8, 8.1) befestigt ist und über mindestens eine Kraftübertragung (11, 6, 5, 5.1) auf Zug belastbar ist. Der Zug wird über ein Seilzugsystem mit integrierter Freilauf- und Verstellfunktion zwischen der Unterkörperanbindung und dem starren Rückenelement 1, das bevorzugt durch eine an die natürliche Form der Wirbelsäule angepasste Struktur sowie Hüft- Bauch- und Oberkörperanbindung in Position gehalten wird, aufgebracht und unterstützt dadurch die Rückenstreckermuskulatur - besonders im Lendenwirbelbereich - in der statischen Haltearbeit und die Hüftstreckermuskulatur in der konzentrischen und exzentrischen Arbeit.

Es ist vorteilhaft, wenn eine Einstellbarkeit der Kraftübertragung mit gleichzeitiger Aktivierung des ersten Energiespeichers sowie Beteiligung der Freilauffunktion zum Ausgleich von unsymmetrischen Kräften möglich ist. Unsymmetrische Kräfte treten auf, wenn bspw. nur ein Bein nach vorne gesetzt wird und dadurch einseitig ein entsprechender Teil-Energiespeicher belastet wird. Dies wird bei der hier beschriebenen Ausführungsform durch eine Einstellung der Arbeitslänge einer als Kraftübertragungselement wirkenden Zugseele im mindestens einen Hüftelement, die den Kräfteeinsatz von der Oberschenkelanbindung über eine Umlenkrolle (6) zum Teil-Energiespeicher verstellt, erzielt.

Es ist auch günstig, wenn zwei derart unabhängig voneinander einstellbare Energiespeicher - ein unterer/erster und ein oberer/zweiter Energiespeicher (21, 21.1 und 2) in der Hebehilfe vorgesehen sind, die das gleiche Freilaufkonzept (Längenaus-gleich der Zugseelen über Rollen) nutzen. Bei einer Ausführungsform, die nachfolgend in den Figuren gezeigt ist, ist der untere/erste Energiespeicher zweigeteilt (21, 21.1) - d.h. eine Hälfte befindet sich an der rechten Oberschenkelanbindung und die andere Hälfte an der linken Oberschenkelanbindung. An den zwei Teil-Energiespeichern ist außerdem jeweils das freie Ende der Zugseele (11) befestigt, sodass diese zwischen Oberschenkelanbindung und Rückenelement beim Beugen angespannt und beim Aufrichten entspannt werden.

Dabei kann bei der erfindungsgemäßen Hebehilfe der obere Energiespeicher 2, dessen Einsatz zur Änderung der Systemcharakteristik führt bzw. die Unterstützungskraft mit einer zweiten zusätzlichen stufenlosen Kraft ergänzt, wahlweise durch entsprechende Verstellung des Verstellriemens 12 aktiviert und konfiguriert werden. Beim Einsatz von zwei voneinander getrennten Energiespeichern (unterer und oberer Energiespeicher) ist es sinnvoll, die jeweiligen Einstellmöglichkeiten voneinander zu trennen, weshalb bei der erfindungsgemäßen Hebehilfe die Größe der Unterstützung durch die unteren/ersten Energiespeicher und die positionsabhängige zusätzliche Kraftunterstützung durch den oberen/zweiten Energiespeicher unabhängig stufenlos über die Umlenkrollenverstellenden Verstellriemen verstellt werden kann.

Als Federelemente eignen sich stufenlose Energiespeicher, wie Metallfedern, Gummizüge, Elastomere, sowie Dämpfer, die Rückstellkräfte gegen Dehnung ausüben können. Es ist außerdem günstig, das elastische Verhalten der Hebehilfe durch Austauschen der jeweiligen Energiespeicher (21, 21.1, 2) verändern zu können. In der erfindungsgemäßen Hebehilfe ermöglicht dementsprechend ein Baukastensystem aus Energiespeichern mit unterschiedlichen Federcharakteristika die Konfiguration des System-verhaltens abhängig der gewünschten Unterstützung.

Es ist günstig, wenn Energiespeicherelemente einer Hebehilfe gegen Gehbewegungen, d.h. leichte asymmetrische Beinbewegungen des Trägers, keine elastische Gegenkraft aufbringen, damit der Träger nicht gestört wird, wenn er keine Hebebewegung durchführt. Die Hebehilfe besitzt dementsprechend bei einer Ausführungsform eine Freilauffunktion der Zugseele 11 über die Umlenkrollen 6 für den ersten/unteren Energiespeicher und eine Freilauffunktion der Zugseele 11.1 über Umlenkrollen 6, 3 für den zweiten/oberen Energiespeicher.

Bekannte Hebehilfen mit Verstellmechanismen im Hüftbereich leiten entstehende Zugkräfte der Energiespeicher in einen Hüftgurt um, wodurch große Teile der unterstützenden Kraft auf den Bauchbereich des Trägers drücken können. Es ist daher sinnvoll, die Verstellung der Energiespeicherelemente in den Hüftelementen kraftfrei vom Hüftgurt zu halten. Dementsprechend beinhaltet die erfindungsgemäße Hebehilfe Hüftelemente mit flexiblen, aber längs inkompressiblen Leitplatten 13, welche die unterstützenden Zugkräfte der Zugseelen von den Energiespeichern an den Leitplatten 13 und das starre Rückenelement 1 angreifen lassen, die somit nicht in den Körper des Trägers eingeleitet werden.

Durch die Verwendung eines starren Rückenelements 1 werden außerdem Kräfte, die zwischen Oberschenkelanbindung und Schulterträgern durch Dehnung der Energiespeicherelemente entstehen und als Zug- und Biegekräfte auf die Wirbelsäule wirken können, aufgenommen und nur auf spezifische Bereiche des Körpers aufgebracht.

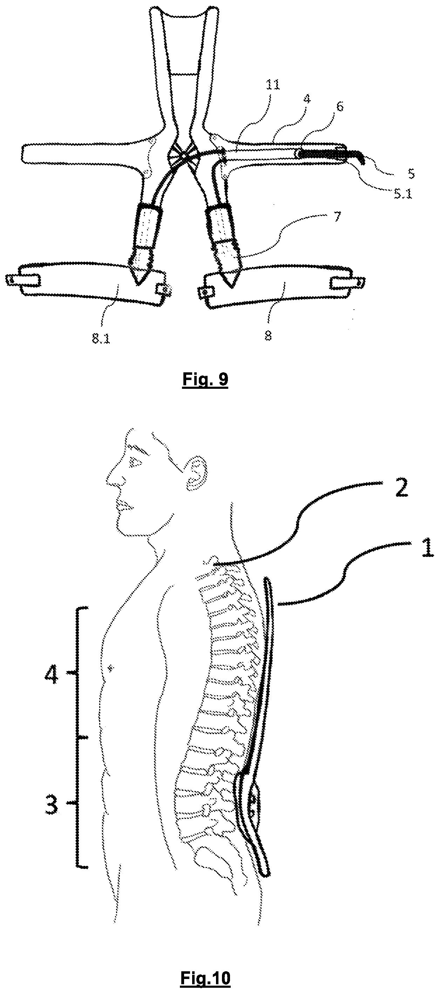

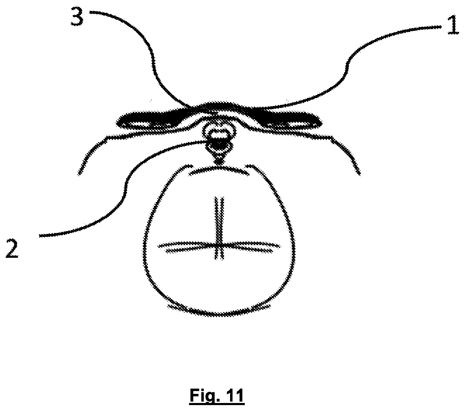

Die Hebehilfe ist aufgrund ihres einfachen Aufbaus leicht und unproblematisch anzulegen und unterstützt die Rückenmuskulatur des Trägers beim Bücken mit Lasten durch Stabilisierung und die Hüftstreckermuskulatur durch unterstützende Zugkräfte beim Heben. Der Träger wird am Heben mit stark gerundetem Rücken im Lendenwirbelbereich gehindert und dadurch schädliche Spitzenlasten auf die Bandscheiben reduziert, indem das Rückenelement Teile der Zugkräfte als Druck auf den unteren Rücken aufbringt und dadurch auch eine Haltungskorrektur vornimmt. Außerdem wird die Rückenstreckermuskulatur des Trägers in der statischen Arbeit unterstützt, wodurch Schmerzen durch Muskelverhärtung vorgebeugt wird (s. Fig. 10 und Fig.11)

Nachfolgend wird die Erfindung anhand von Ausführungsbeispielen sowie der Zeichnung näher erläutert, auf die sie jedoch keinesfalls eingeschränkt ist. Darin zeigen:
Fig. 1 perspektivisch eine von einer Person getragene Hebehilfe in Rückansicht
Fig. 2 schematisch Einzelelemente der Hebehilfe mit elastischen Energiespeichern in nicht angelegtem Zustand aus der Rückansicht.
Fig. 3 schematisch die Einzelelemente der Fig. 2 bei Aktivierung und Verstellung der unteren Teil-Energiespeicher
Fig. 4 schematisch die Einzelelemente aus Fig. 2 und Fig. 3 bei Aktivierung und Verstellung des zweiten Energiespeichers;
Fig. 5: schematisch eine perspektive Darstellung eines Trägers mit Hebehilfe unter Nutzung der Freilauffunktion beim Laufen;
Fig. 6 schematisch einige statische Arbeitspositionen;
Fig. 7 schematisch Einzelelemente der Hebehilfe beim Schreiten sowie eine Rückansicht des Trägers;
Fig. 8 eine Detailansicht des Hüftmodul-/Bauchgurtbereichs mit unteren Teil-Energiespeichern der Hebehilfe; und
Fig. 9 eine Rückansicht einer einfachen Ausführungsform der Hebehilfe mit unteren Teil-Energiespeichern
Fig. 10 eine schematische Ansicht der Wirbelsäule mit aufliegendem Rückenelement; und
Fig.11 eine schematische Ansicht eines Querschnitts durch den Rumpf mit einem Wirbelkörper mit aufliegendem Rückenelement

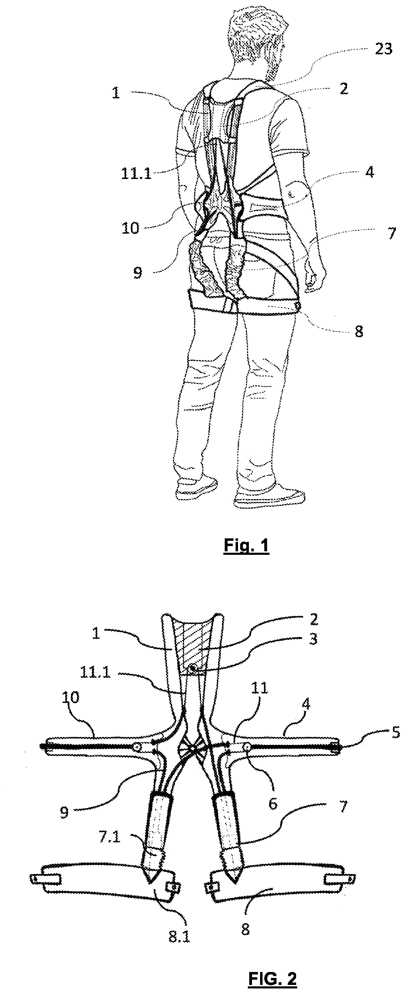

Die Hebehilfe **(****Fig. 1****)** mit Schulterträgern als Oberkörperanbindung 23, zweiteiligem Bauchgurt 17 und Unterkörperanbindung (hier Oberschenkelanbindungen (8, 8.1), mit einem dazwischen am Körper angeordneten starren Rückenelement 1 und Hüftelementen 10, 4, Energiespeichern 21 und Kraftübertragungen (hier Zugseelen 11, 11.1) steuert die Krafteinwirkung der Energiespeicher und soll den Träger in seiner Haltung beim Heben und durch elastische Federkraft in der Hebebewegung unterstüt-zen. Die Unterstützung erfolgt durch direkte Haltungskorrektur gegen Rundrücken und schräges Heben sowie eine Unterstützung per Zugkraft abhängig vom Beugewinkel der Hüfte durch stufenlose Energiespeicher - hier Federelemente 21, 21.1, 2.

Durch Beugen (respektive Verkleinerung des Hüftbeugewinkels) verlängert sich der Weg der Zugseelen 11, 1 1.1 in den Verbindungsmänteln 7,7.1 zwischen Rückenelement 1 und Oberschenkelanbindungen 8, 8.1, welche die Energiespeicher auf Dehnung beanspruchen und gleichzeitig Rückstellkräfte aufbauen.

In der Ausführungsform der Fig. 1 - 8 sind ein geteilter unterer/ erster Energiespeicher 21, 21.1, sowie ein oberer/ zweiter Energiespeicher 2 verbaut. Diese können mit Elastomeren, Federn und anderen Materialien, die eine elastische Rückstellkraft bei Dehnung ausüben, ausgerüstet sein, wobei auch eine Kombination von Federelementen möglich ist, die bei Zugeinwirkung proportional zur Dehnung stufenlos eine Zugkraft aufbringen.

Das starre Rückenelement 1 der Hebehilfe, welches die Hüftelemente 4, 10 und die Energiespeicher 21, 21.1 und 2 verbindet sowie die Zugseele(n) 11, 11.1 leitet, ist in sich stabil. Es nimmt Kräfte der Zugseelen und der Umlenkrollen 6 bei Verstellung auf den Leitplatten 13 in den Hüftelementen 4, 10 und der oberen Freilaufrolle 3 auf und leitet diese ins Material ab. Ein Teil der Kräfte wirkt als haltungskorrigierender Druck auf dem unteren Rücken.

Die Hebehilfe (Fig. 2) beinhaltet zwei getrennte Energiespeicher - einen oberen Energiespeicher (2) und einen unteren (geteilten) Energiespeicher (21, 21.1). Beide Energiespeicher sind stufenlos verstellbar über die Freilaufrolle 3 für die Zugseele 11.1 und eine Freilaufrolle 6 für die Zugseele 11.

Das untere/ erste Energiespeichersystem besteht aus dem rechten und linken Teilspeicher (21, 21.1), die an der rechten, bzw. linken Oberschenkelanbindung (8, 8.1) fixiert ist, der an den Teilspeichern angebundenen Zugseele 11 und der Verstell- und Freilaufeinrichtung im rechten Hüftelement 4, bestehend aus einer Umlenkrolle 6, dem Verstellriemen 5 und der verstellbaren Klemmeinrichtung 5.1.

Der obere/ zweite elastische Energiespeicher 2 ist mit der Freilaufrolle 3 verbunden, wobei die zweite Zugseele 11.1, die an den Anbindungspunkten 22, 22.1 an den Oberschenkelanbindungen 8, 8.1 fixiert ist, an der Freilaufrolle 3 umgelenkt wird und diese entgegen der Federkrafut des zweiten Energiespeichers nach unten zieht.

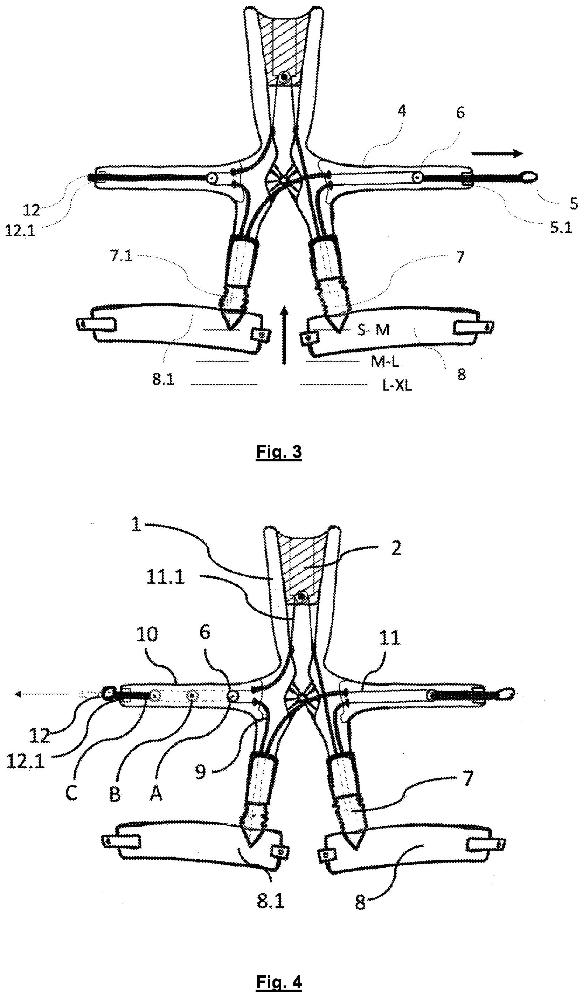

Fig. 3 zeigt eine **Änderung** der Unterstützung durch das erste Energiespeichersystem. Dies erfolgt durch Zug am im rechten Hüftelement geführten Verstellriemen 5, wodurch sich dieser weiter durch die geöffnete Klemmeinrichtung 5.1 bewegt. Anschließend wird durch Schließen der Klemmeinrichtung 5.1 der Verstellriemen 5 in der Position fixiert. Die Verstellung erfolgt stufenlos, kann jedoch auch durch eine herkömmliche Schnalle mit Dorn in definierten Einstellstufen umgesetzt werden. Durch das Bewegen des Verstellriemens 5 wird die auf der Leitplatte 13 in der Führungsauskleidung 14 geführte Freilaufrolle 6 verschoben und damit die wirksame Länge der Zugseele 11 zwischen Oberschenkelanbindungen 8, 8.1 und Rückenelement 1 verkürzt. Die Führungsauskleidung 14 bewirkt eine Reduktion der Reibung zwischen Zugseele 11 und dem Mantel 15 des rechten Hüftelements 4, der das Zugseilsystem dort einhüllt und zusammenhält. Die Verkürzung der wirksamen Länge der Zugseele 11 bewirkt eine stärkere und schon ab einem kleineren Hüftwinkel < 180° (dies entspricht dem aufrechten Stehen) zu wirken beginnende Unterstützungskraft. Unabhängig von der Unterstützungskrafteinstellung des ersten elastischen Speichers 21, 21.1 wird die Freilauffunktion über die obere Freilaufrolle 3 aufrechterhalten. Durch die Energiespeicher 7, 7.1 aufgebrachten Zugkräfte werden nicht auf den Bauchgurt 17 übertragen, sondern bei Schließen des Bauchgurt-verschlusses entlang der in Längsrichtung inkompressiblen Leitplatte 13 in das Material des starren Rückenelements 1 eingeleitet.

Das selbe Funktionsprinzip gilt für die Einstellung des zweiten Energiespeichers 2, dessen Wirkung ebenfalls stufenlos über den Gurt 12 im linken Hüftelement 10 und - bei dieser Ausführungsform - eine Klemmeinrichtung 12.1 verstellbar ist (Fig .4). Der Verlauf der zweiten Zugseele 11.1 über die Umlenkrolle 6 im Hüftelement 10 und die obere Freilaufrolle 3 ermöglicht die Freilauffunktion unabhängig von der Unterstützungskrafteinstellung.

Selbstverständlich können auch Schnallen mit Dorn für die Einstellung und Festlegung der auf der Leitplatte 13 geführten Umlenkrolle 6 für die zweiten Zugseele 11.1 anstelle einer Klemmeinrichtung verwendet werden.

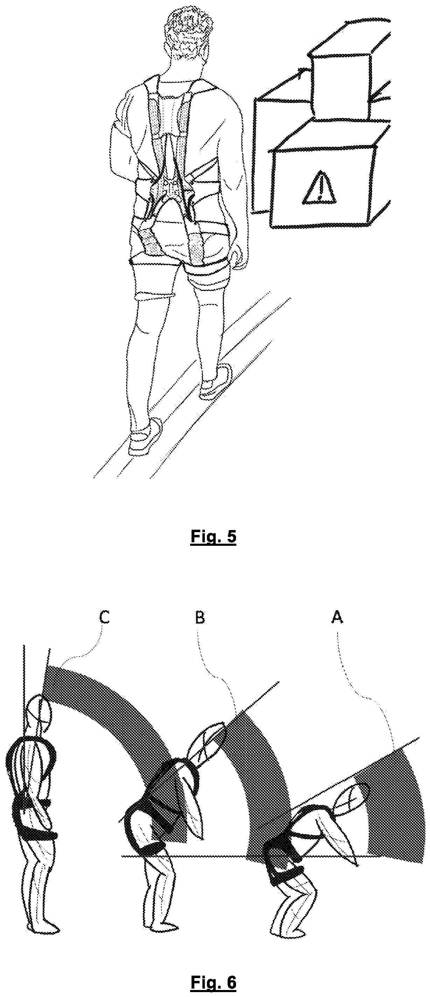

In Fig. 6 sind beispielhaft unterschiedliche Einstellungen in Bezug auf die Verstellung des zweiten Energiespeichers 2 gezeigt, welche eine Änderung der **Unterstützungskraft und deren** Einwirkung in Abhängigkeit vom Beugewinkel bewirken. D.h. je nach Position der Umlenkrolle 6 im linken Hüftmodul 10 wirkt die Unterstützungskraft des zweiten Unterstützungselements 2 früher (starke Unterstützung, Beispiel C) oder später (schwache Unterstützung, Beispiel A) beim Beugen.

Die Einstellbarkeit eines zusätzlichen und getrennt von anderen Energiespeichern (21, 21.1) vorgesehenen Energiespeichers 2 ist einzigartig und eröffnet dem Träger viele zusätzliche Einstellmöglichkeiten durch Kombination der beiden Energiespeichersysteme. So kann die komplette Systemcharakteristik verändert werden. Die zusätzliche Unterstützungskraft des zweiten Energiespeichers 2 wird zur Grundunterstützung des ersten Energiespeichersystems durch die elastischen Energiespeicher 21, 21.1 addiert. Durch die individuelle Einstellung und die zusätzliche Aktivierung kann der Träger das System passend zur eigenen Vorliebe und des Arbeitsumfeldes anpassen. Somit wird ein hoher Trage- und Anwendungskomfort ermöglicht.

Die Unterstützungskrafteinstellung der beiden Energiespeicher über nur je einen Verstellriemen 5,12 und die dazugehörige Klemmeinrichtung 5.1, 12.1 ermöglicht ein schnelles und einfaches Einstellen vor und zwischen Hebetätigkeiten. Die Einstellung erfolgt für das jeweilige Energiespeichersystem symmetrisch für die linke und rechte Körperhälfte, um asymmetrische Belastungen durch die Hebehilfe auf den Körper zu verhindern. Zum Abschalten/Lösen der Unterstützung durch die Energiespeicher ist die rechte Klemmeinrichtung 5.1 für den ersten Energiespeicher 21, 21.1 und die linke Klemmeinrichtung 12.1 für den zweite Energiespeicher 2 verantwortlich und kann einfach verstellt und gelöst werden.

In Fig. 5 ist ersichtlich, dass durch die Gehbewegung einseitige Hüftwinkeländerungen stattfinden, die sich auf die Energiespeicher 21, 21.1 in den Verbindungsmänteln 7, 7.1 auswirken. Ohne eine Freilaufeinrichtung 6 wirkten sich diese als Dehnung auf die Energiespeicher 7, 7.1 aus, die dann eine unsymmetrische Zugkraft aufbringen und so die Laufbewegung einschränken würden.

Das erfindungsgemäße System mit oberem und unterem Energiespeicher ermöglicht die Einstellung zweier getrennt voneinander aktivierbarer Unterstützungen. Falls diese beim Laufen immer deaktiviert werden müssten, würde die Akzeptanz bei Trägern stark sinken.

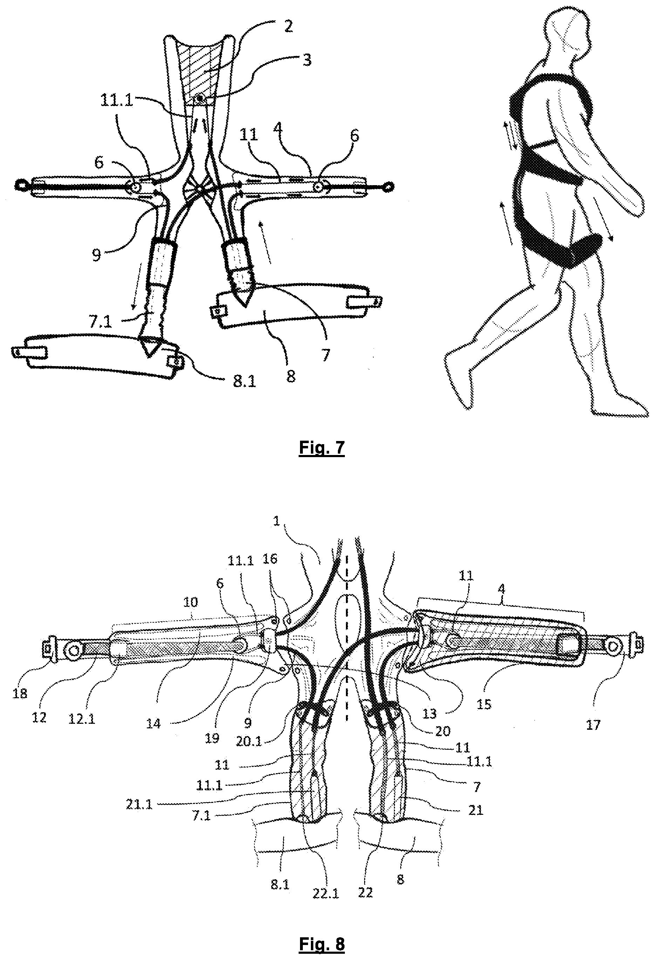

Deshalb beinhaltet die Hebehilfe (Fig. 7) eine integrierte Freilauffunktion, die unabhängig von jeder Einstellung im System ist, da über Umlenkrollen 6, 3 die Zugseelen 11, 11.1 beim Laufen entsprechend verstellt und ausgeglichen werden. Die Freilauffunktion wird durch die asymmetrische Bewegung der Beine, bspw. bei einer Laufbewegung aktiv, während sie bei symmetrischem Beugen inaktiv bleibt, wodurch die Energiespeichersysteme Unterstützungskraft aufbringen können. Das gesamte Funktionsprinzip ist mechanisch und benötigt keinerlei elektrische Steuerung. Die sich durch die Freilauffunktion durch das System bewegenden Zugseelen 11, 11.1 sind in den Führungsmänteln 9 mit reibungsarmen Führungsauskleidung 14 in den Hüftele-menten 4, 10 auf ebenfalls reibungsarmen Leitplatten 13 geführt. Dadurch wird der Träger nur minimal beeinflußt.

Die Zugseelen 11, 11.1 sind in Leitmänteln 9 auf in Längsrichtung inkompressiblen, aber flexiblen Leitplatten 13 reibungsarm geführt und werden über die Umlenkrollen 6 und 3 umgelenkt. Dadurch bleiben die Zugseelen 11, 11.1 flexibel. Die Führungsauskleidung 14 schwächt die Reibung der Zugseelen durch eine spezielle Materialabstimmung auf den Leitplatten und dem Leitmantel ab, so dass sie fast reibungsfrei sind. Dafür eigenen sich bspw. Bürsten oder reibungsarme Kunststoff-Futter. Bewegt der Träger ein Bein, bewegen sich auch alle mit einem Ende an den Oberschenkelanbindungen 8, 8.1 befestigte Zugseelen 11,11' im gesamten System. Dies verringert die Einschränkung des Trägers durch den Zugseelenzug beim Gehen auf eine nicht wahrnehmbare Größe.

In Fig. 9 ist eine einfachere Ausführungsform der Erfindung gezeigt, die nur über eine Zugseele 11 und nur eine Verstellbarkeit im rechten Hüftmodul 4 mit Freilaufrolle 6, Verstellriemen 5 und verstellbarer Klemmeinrichtung 5.1 verfügt. Der zweite Energiespeicher ist nicht vorhanden, wodurch das linke Hüftelement keine einstellende Funktion besitzt. Das System hat die Funktionen des ersten geteilten Energiespeichers 21, 21.1, d.h. eine Freilauffunktion und die Haltungs- und Bewegungsunterstützung.

Fig. 10 zeigt die Auflage des Rückenelements im Bereich der Lendenwirbelsäuel bis zu den ersten Rippen und dem Kreuzbein. In diesem Bereich übt das am Körper befestigte Rückenelement Rückstellkräfte gegen eine Beugung des Rumpfs aus und verhindert somit einen Rundrücken. Insbesondere da das relativ starre Rückenelement bevorzugt eine Vertiefung zur Aufnahme der Dornfortsätze der Wirbelkörper aufweist, ist es an seitlicher Verschiebung gehindert und wirkt somit auch hindernd auf eine bandscheibenbelastende Seitwärtsbewegung der Wirbelsäure, wie in Fig. 11 gezeigt, dass ein ein der optimalen Wirbelsäulenhaltung entsprechendes Formteil, dessen Mindestlänge vom Lendenwirbel 2 oder 3 bis Brustwirbel 7 beträgt, maximal dem Abstand vom Kreuzbein bis Halswirbel 3 beträgt und dessen Breite zwischen der Kreuzbeinbreite bis zur Rückenbreite variieren kann, mit einer Längsvertiefung zur Aufnahme der Dornfortsätze der Wirbelsäule in dessen dem Rücken zugewandten Seite, und Befestigungseinrichtungen für Tragegurte zur Befestigung des Rückenelements an einer Person.

Die Rückenelemente sind generell zur Rückenschulung bzw. Verhinde-rung von Fehlbewegungen geeignet, um einseitige Belastungen einzuschränken und besonders belastende Bewegungen beim Heben zu vermeiden.

Typische Biegemodul-Werte für ein Rückenelementmaterial liegen zwischen 500 - 100000Mpa, bevorzugt zwischen 800 bis 5000 und besonders bevorzugt zwischen 1500 und 3000 mPa. Geeignet können Schichtmaterialen, wie Holzlaminate oder aus Schichten aufgebaute Kompositmaterialien, Kunststoffe aber auch Metallplatten - bspw. aus Aluminium - sein.

### Bezugszeichenliste

1 Rückenelement
2 zweiter elastischer Energiespeicher
3 Freilaufrolle
4 Hüftelement rechts
5 Verstellriemen von 4
5.1 Verstellbare Klemmeinrichtung von 4
6 Umlenkrolle/Freilaufrolle im Hüftelement 4, 10
7 Rechter Verbindungsmantel für Zugseelen (11,11.1) und Federelement (21) zwischen Rückenelement 1 und rechter Oberschenkelanbindung (8)
7.1 Linker Verbindungsmantel für Zugseelen (11,11.1) und Federelement (21.1) zwischen Rückenelement 1 und linker Oberschenkelanbindung (8.1)
8 rechte Oberschenkelanbindung
8.1 linke Oberschenkelanbindung
9 Führungsmantel der Zugseele(n) 11, 11.1
10 Hüftelement links
11 erste Zugseele des ersten Energiespeichers
11.1 zweite Zugseele des zweiten Energiespeichers
12 Verstellriemen in 10
12.1 verstellbare Klemmeinrichtung in 10
13 Leitplatte(n) in Hüftelement(en) für Seele(n) (11, 11.1)
14 Führungsauskleidung in 15
15 Mantel für Hüftelemente 4, 10
16 Befestigungspunkt von 13 an 1
17 Bauchgurt in 4
18 Bauchgurtverschluss an 17
21 rechter Energiespeicher in 7
21.1 linker Energiespeicher in 7.1
22 Anbindungspunkt der Zugseele 11.1 an 8
22.1 Anbindungspunkt für Zugseele 11.1 an 8.1
23 Oberkörperanbindung (Schulterträger)
A Einstellposition für Unterstützung für tiefes Beugen
B Einstellposition für Unterstützung bei mittlerem Beugen
C Einstellposition für Unterstützung ab leichter Beuge

## Patentansprüche

1. Mechanische Hebehilfe zur Unterstützung der Rumpfmuskulatur, die über eine obere Oberkörperanbindung (23) und eine untere Unterkörperanbindung mit zwei Oberschenkelanbindungen (8, 8.1) ortsfest auf dem Rücken einer tragenden Person anordenbar ist, mit - einem unteren zweiteiligen mechanischen Energiespeicher für Spannenergie mit zwei elastischen Teil-Energiespeichern (21, 21.1) mit Federelemente zwischen der jeweiligen Oberschenkelanbindung (8, 8.1) und einem festen Rückenelement (1), wobei die Federelemente mit einem Ende an jeweils einer der Oberschenkelanbindungen (8,8.1) und am anderen Ende jeweils an mindestens einem Zugelement (11, 11.1) befestigt sind,
wobei das Rückenelement (1) am Rücken befestigt ist und Kräfte von den elastischen Teil-Energiespeichern (21, 21.1) über das mindestens eine Zugelement (11, 11.1) aufnimmt, umverteilt und ableitet,
- wobei das mindestens eine Zugelement (11, 11.1) den jeweiligen elastischen Teil-Energiespeicher (21, 21.1) mit dem Rückenelement (1) (1) verbindet,
wobei ein rechtes (4) und ein linkes (10) Hüftelement mit verstellbaren Freilaufeinrichtungen mit jeweils einer Freilaufrolle (6) für das mindestens eine Zugelement (11, 11.1) in mindestens einem der Hüftelemente (4, 10) am festen Rückenelement (1) angelenkt sind und wobei mindestens eine verstellbare Klemmeinrichtung (5.1, 12.1) in den Hüftelementen (4, 10) die Aktivierung des unteren zweiteiligen mechanischen Energiespeichers ändert mittels der jeweiligen Freilaufrolle (6), die über eine stufenlose Verstellung mindestens eines Verstellriemens (5, 12) verschieblich ist und so die wirksame Länge des mindestens einen Zugelementes (11, 11.1) ändert.

2. Hebehilfe nach Anspruch 1, ferner **gekennzeichnet durch** einen unabhängig vom unteren zweiteiligen mechanischen Energiespeicher einstellbaren oberei elastischen Energiespeicher (2) mit einer daran befestigten oberen Freilaufrolle (3), der über ein zweites Zugelement (11.1) mit den beiden Oberschenkelanbindungen (8, 8.1) verbunden und aktivierbar ist, wobei das feste Rückenelement (1) Kräfte von den zwei elastischen Teil-Energiespeichern (21, 21.1) und oberen elastischen Energiespeicher (2) aufnimmt, umverteilt sowie an spezifische Stellen ableitet.

3. Hebehilfe nach einem der vorangehenden Ansprüche, **dadurch gekennzeichnet, dass** mindestens einer der zwei elastischen Teil-Energiespeicher (21, 21.1) oder obere elastische Energiespeicher (2) stufenlos Kraft aufnimmt und abgibt.

4. Hebehilfe nach einem der vorangehenden Ansprüche, **gekennzeichnet durch** zwei voneinander getrennte Verstellriemen (5, 12) in den Hüftelementen (4,10) zur Einstellung des Unterstützungsgrades des unteren zweiteiligen mechanischen Energiespeichers und des oberen elastischen Energiespeichers (2) durch Verkürzung der wirksamen Länge der jeweiligen Zugelemente (11,11.1) über Verschiebung der jeweiligen Freilaufrollen (6) .

5. Verwendung der Hebehilfe nach einem der vorangehenden Ansprüche, als medizinische Hebehilfe für Pflegende oder Bauarbeiter oder Umzugsarbeiter - bzw. Logistikarbeiter-Trageunterstützung.

6. Hebehilfe nach einem der Ansprüche 1 bis 4, **dadurch gekennzeichnet, dass** das feste Rückenelement (1) zur Haltungsunterstützung ein der optimalen Wirbelsäulenhaltung entsprechendes Formteil ist, dessen Mindestlänge vom Lendenwirbel 2 oder 3 bis Brustwirbel 7, maximal dem Abstand vom Kreuzbein bis Halswirbel 3 beträgt und dessen Breite zwischen der Kreuzbeinbreite bis zur Rückenbreite variieren kann; mit einer Längsvertiefung zur Aufnahme der Dornfortsätze der Wirbelsäule in dessen dem Rücken zugewandten Seite, und Befestigungseinrichtungen für Tragegurte zur Befestigung des Rückenelements an einer Person aufweist, das Rückstellkräfte gegen eine Beugung des Rumpfs ausübt und einen gerundeten Rücken vermeidet, und eine Seitwärtsbewegung der Wirbelsäule hindert.

7. Hebehilfe nach einem der Ansprüche 1 bis 4 oder 6, **dadurch gekennzeichnet, dass** das feste Rückenelement (1) einen Biegemodul von 500 bis 80 000 Mpa, bevorzugt 1000 - 50 000 Mpa und besonders bevorzugt zwischen 1500 und 10 000 Mpa aufweist.

8. Hebehilfe nach einem der Ansprüche 1 bis 4, 6 oder 7, **dadurch gekennzeichnet, dass** das feste Rückenelement (1) im wesentlichen ein Kunststoffteil, auch faserverstärkten Kunststoff, Laminat, auch Holzlaminat oder Schichtmaterial oder eine Metall-Legierung, ggf. auch in einem Schichtmaterial, aufweist.

## Claims

1. Mechanical lifting aid for supporting the trunk muscles, which can be arranged in a fixed position on the back of a person carrying it via an upper body connection (23) and a lower body connection with two thigh connections (8, 8.1), with a lower two-part mechanical energy storage device for tensioning energy with two elastic partial energy storage devices (21, 21.1) with spring elements between the respective thigh connection (8, 8.1) and a strong back element (1), wherein the spring elements are attached at one end to one of the thigh connections (8,8.1) and at the other end to at least one tension element (11, 11.1),
wherein the back element (1) is attached to the back and absorbs, redistributes and dissipates forces from the elastic partial energy storage devices (21, 21.1) via the at least one tension element (11, 11.1),
- wherein the at least one tension element (11, 11.1) connects the respective elastic partial energy storage device (21, 21.1) to the back element (1) (1),
wherein a right (4) and a left (10) hip element with adjustable freewheel devices, each with a freewheel roller (6) for the at least one tension element (11, 11.1) in at least one of the hip elements (4, 10) are articulated on the strong back element (1), and wherein at least one adjustable clamping device (5.1, 12.1) in the hip elements (4, 10) changes the activation of the lower two-part mechanical energy storage device by means of the respective freewheel roller (6), which is displaceable via a continuous adjustment of at least one adjustment strap (5, 12) and thus changes the effective length of the at least one tension element (11, 11.1).

2. Lifting aid according to claim 1, further **characterized by**
an upper elastic energy storage device (2) which can be adjusted independently of the lower two-part mechanical energy storage device and has an upper freewheel roller (3) attached thereto, which is connected to the two thigh connections (8, 8.1) via a second tension element (11.1) and can be activated, wherein the strong back element (1) absorbs forces from the two elastic partial energy storage devices (21, 21.1) and upper elastic energy storage device (2), redistributes them and diverts them to specific locations.

3. Lifting aid according to one of the preceding claims, **characterized in that**
at least one of the two elastic partial energy storage devices (21, 21.1) or upper elastic energy storage devices (2) continuously absorbs and releases force.

4. Lifting aid according to one of the preceding claims, **characterized by** two separate adjustment straps (5, 12) in the hip elements (4,10) for adjusting the degree of support of the lower two-part mechanical energy storage device and the upper elastic energy storage device (2) by shortening the effective length of the respective tension elements (11, 11.1) by shifting the respective freewheel rollers (6).

5. Use of the lifting aid according to one of the preceding claims, as a medical lifting aid for caregivers or construction workers or moving workers - or logistics worker carrying support.

6. Lifting aid according to one of claims 1 to 4, **characterized in that**
the strong back element (1) for posture support is a shaped part corresponding to the optimum spinal posture, the minimum length of which is from the lumbar vertebra 2 or 3 to the thoracic vertebra 7, a maximum of the distance from the sacrum to the cervical vertebra 3 and the width of which can vary between the width of the sacrum and the width of the back; with a longitudinal recess for receiving the spinous processes of the spine in its side facing the back, and attachment devices for the carrying belts for attaching the back element on a person, which exerts restoring forces against bending of the torso and avoids a rounded back, and prevents sideways movement of the spine.

7. Lifting aid according to one of claims 1 to 4 or 6, **characterized in that** the strong back element (1) has a flexural modulus of 500 to 80 000 Mpa, preferably 1000 - 50 000 Mpa and particularly preferably between 1500 and 10 000 Mpa.

8. Lifting aid according to one of claims 1 to 4, 6 or 7, **characterized in that** the strong back element (1) essentially comprises a plastic part, also fiber-reinforced plastic, laminate, also wood laminate or layered material or a metal alloy, optionally also in a layered material.

## Revendications

1. Aide mécanique au levage pour soutenir les muscles du tronc, qui peut être placée en position fixe sur le dos d'une personne qui la porte via une connexion supérieure du corps (23) et une connexion inférieure du corps avec deux connexions de cuisse (8, 8.1), avec un dispositif d'accumulation d'énergie mécanique inférieur en deux parties pour tendre l'énergie avec deux dispositifs d'accumulation d'énergie partiels élastiques (21, 21.1) avec des éléments de ressort entre la connexion de cuisse respective (8, 8.1) et un élément dorsal solide (1), dans lequel les éléments de ressort sont attachés à une extrémité à l'une des connexions de cuisse (8,8.1) et à l'autre extrémité à au moins un élément de tension (11, 11.1),
dans lequel l'élément dorsal (1) est attaché au dos et absorbe, redistribue et dissipe les forces provenant des dispositifs élastiques de stockage partiel d'énergie (21, 21.1) par le biais d'au moins un élément de tension (11, 11.1),
- dans lequel au moins un élément de tension (11, 11.1) relie le dispositif de stockage partiel d'énergie élastique respectif (21, 21.1) à l'élément dorsal (1) (1),
dans lequel un élément de hanche droit (4) et gauche (10) avec des dispositifs de roue libre réglables, chacun avec un rouleau de roue libre (6) pour au moins un élément de tension (11, 11.1) dans au moins un des éléments de hanche (4, 10) sont articulés sur l'élément dorsal solide (1), et dans lequel au moins un dispositif de serrage réglable (5.1, 12.1) dans les éléments de hanche (4, 10) modifie l'activation du dispositif de stockage d'énergie mécanique inférieur en deux parties au moyen du rouleau à roue libre respectif (6), qui peut être déplacé par un réglage continu d'au moins une sangle de réglage (5, 12) et modifie ainsi la longueur effective d'au moins un élément de tension (11, 11.1).

2. Aide au levage selon la revendication 1, **caractérisée en outre par**
un dispositif de stockage d'énergie élastique supérieur (2) qui peut être réglé indépendamment du dispositif de stockage d'énergie mécanique inférieur en deux parties et qui comprend un rouleau supérieur de roue libre (3) attaché à celui-ci, qui est relié aux deux raccords de cuisse (8, 8.1) par un deuxième élément de tension (11.1) et est activé, dans lequel l'élément dorsal solide (1) absorbe les forces provenant des deux dispositifs de stockage d'énergie partiels élastiques (21, 21.1) et du dispositif de stockage d'énergie élastique supérieur (2), les redistribue et les détourne vers des endroits spécifiques.

3. Aide au levage selon l'une des revendications précédentes, **caractérisée en ce que**
au moins l'un des deux dispositifs de stockage partiel d'énergie élastique (21, 21.1) ou des dispositifs de stockage d'énergie élastique supérieurs (2) continuellement absorbe et libère la force.

4. Aide au levage selon l'une des revendications précédentes, **caractérisée par** deux sangles de réglage séparées (5, 12) dans les éléments de hanche (4,10) pour ajuster le degré de soutien du dispositif de stockage d'énergie mécanique inférieur en deux parties et du dispositif de stockage d'énergie élastique supérieur (2) en raccourcissant la longueur effective des éléments de tension respectifs (11, 11.1) en déplaçant les rouleaux de roue libre respectifs (6).

5. Utilisation de l'aide au levage selon l'une des revendications précédentes, comme aide au levage médical pour les soignants ou les ouvriers du bâtiment ou les déménageurs - ou aide au portage pour les logisticiens.

6. Aide au levage selon l'une des revendications 1 à 4, **caractérisée en ce que**
l'élément dorsal solide (1) pour le soutien de la posture est une pièce de forme correspondant à la posture optimale de la colonne vertébrale, dont la longueur minimale va de la vertèbre lombaire 2 ou 3 à la vertèbre thoracique 7, dont la distance maximale va du sacrum à la vertèbre cervicale 3 et dont la largeur peut varier entre la largeur du sacrum et la largeur du dos ; avec une évidement longitudinal destiné à recevoir les apophyses épineuses de la colonne vertébrale dans sa partie tournée vers le dos, et des dispositifs d'attache pour les ceintures de transport permettant de solider l'élément dorsal sur une personne, qui exerce des forces de rappel contre la flexion du torse et évite un dos arrondi, et empêche les mouvements latéraux de la colonne vertébrale.

7. Aide au levage selon l'une des revendications 1 à 4 ou 6, **caractérisée en ce que** l'élément dorsal solide (1) a un module de flexion compris entre 500 à 80 000 Mpa, de préférence entre 1000 - 50 000 Mpa et de préférence encore entre 1500 et 10 000 Mpa.

8. Aide au levage selon l'une des revendications 1 à 4, 6 ou 7, **caractérisée en ce que** l'élément dorsal solide (1) comprend essentiellement une pièce en plastique, également en plastique renforcé de fibres, un stratifié, également en bois stratifié ou en matériau stratifié, ou un alliage métallique, éventuellement aussi en matériau stratifié.
